# EUROPEAN PATENT SPECIFICATION

(11) **EP 1 323 432 B1**
(45) Date of publication and mention of the grant of the patent: **24.08.2005**
(21) Application number: 01830818.9
(22) Date of filing: 28.12.2001
(51) Int. Cl.: A61K 47/48, A61K 31/505, A61P 7/04

(54) **Complexes of cyclodextrins with potassium ion homeostasis regulators**
Zyklodextrin-Komplexe mit Kalium Ion Homeostasis Regulatoren
Complexes de cyclodextrines pour le traitement des troubles de l'homéostase du potasse

(43) Date of publication of application: 02.07.2003
(73) Proprietor: Cosci, Sandra, (Prov. of Lucca) (IT)
(72) Inventor: Cosci, Sandra, (Prov. of Lucca) (IT)
(74) Representative: Modiano, Guido

(56) References cited:
- EP-A- 0 579 435
- WO-A-99/36072
- AMMAR H O ET AL: "CYCLODEXTRINS IN ACETAZOLAMIDE EYE DROP FORMULATIONS" PHARMAZIE, VEB VERLAG VOLK UND GESUNDHEIT. BERLIN, DD, vol. 53, no. 8, 1 August 1998 (1998-08-01), pages 559-562, XP000769915 ISSN: 0031-7144

## Description

### Technical Field

The present invention relates to complexes of cyclodextrins with substances of the class of pyridine-3-oxides, thiazides and cyanoguanidines. These complexes are useful in the treatment of disorders characterized by muscular weakness and muscular fatigue observed in healthy individuals subjected to prolonged and/or intense physical exercise, in overweight and obese individuals after physical exercise, in individuals suffering from primary and secondary periodic paralyses, in mitochondrial myopathies, in myotonias, and whenever an uncontrolled loss of blood potassium is observed.

### Background Art

Pyridine-3-oxide derivatives and cyanoguanidines have been known for many years for their antihypertensive effects. Both classes of substances owe their clinical use to their powerful vasodilatory effect on peripheral arteries of muscles, of the skin and of the gastrointestinal region. These arteriolar vasodilators are also capable of improving muscle performance in patients with cardiac failure. This effect is due to an improvement in the oxygenation of peripheral tissues induced by the drugs during muscular exercise, which is well-known to induce compression of muscle arteries with a peripheral ischemia that is even more conspicuous in individuals with reduced peripheral circulation, such as obese individuals. In many cases, after short-term (1-3 months) treatment with 6-[1-piperidinyl]pyrimidine-2,4-diamine-3-oxide, an increase in cardiac output and flow rate has been observed without an increase in heart rate. Long-term (3-4 months or more) chronic treatment with direct vasodilatory drugs, however, can lead to undesirable effects particularly in cardiopathic individuals, such as tachycardia caused by the high adrenergic tone produced by hypotension, water retention and hypertrichosis, which limit their use in these patients (Hardman, Limbird, Molinoff, Ruddon and Goodman, in *Le Basi Farmacologiche della Terapia*, p. 767, 2000). Conspicuous peripheral vasodilatory effects are also observed with thiazides, which are known to be powerful inhibitors of carbonic anhydrase and have therefore been used as diuretics. Currently, thiazides are used most widely in the treatment of disorders of the central nervous system, such as certain genetic forms of juvenile epilepsy, episodic ataxia, in manic states, in the treatment of disorders of the eye region, such as glaucoma, and in the treatment of mountain sickness (Hardman, Limbird, Molinoff, Ruddon and Goodman, in *Le Basi Farmacologiche della Terapia,* p. 677, 2000).

It is known from PCT WO 99/36072 that pyridine-3-oxide derivatives in noncomplexed form can be used in the treatment of neuromuscular disorders, such as muscular paralyses associated with alterations in the levels of blood K⁺. In animal models, 6-[1-piperidinyl]pyrimidine-2,4-diamine-3-oxide, which is the parent of the class, at doses of 1.5-5 mg/day, is capable of preventing muscular weakness and muscular invalidity. Also cyanoguanidines and thiazides are known for the same purpose: [±]-N-cyano-N-4-pyridinyl-N-[1,2,2-trimethylpropyl]-guanidine at 25 mg/day or more is capable of reducing muscular weakness and the frequency of paralysis attacks in patients affected by hypokalemias. Thiazides such as N-[5-sulfamoyl-1,3,4-thiadiazol-2-yl]acetamide, in doses of 100 and 500 mg/day, have been found useful in reducing symptoms and muscular invalidity caused by exercise, which is observed in patients affected by hypokalemias and hyperkalemias. N-[5-sulfamoyl-1,3,4-thiadiazol-2-yl]acetamide is capable of improving muscular performance particularly during anaerobic effort, reducing the blood levels of lactic acid after effort.

In patients and in animal models, these substances are capable of repolarizing muscle fibers and of restoring blood levels of K⁺, which after exercise drop from 4 mEq/l - 5 mEq/l to 0.5 mEq/l - 2 mEq/l for many hours or days, causing permanent invalidity. The therapeutic effects of the three molecules appear 7-10 days after first oral administration and are reversible 3-4 days after suspension of treatment. One of the possible mechanisms by which these substances act is the activation of K⁺ channels of the muscle, restoring the blood levels of this ion.

The disadvantages of the therapeutic use of these molecules in noncomplexed form are their side effects, such as for example tachycardia and hypotension, which prevent or limit their chronic use in the above described muscular disorders. Central effects, such as mental confusion and bland sedation, can be observed at high doses and for chronic treatments in particular with N-[5-sulfamoyl-1,3,4-thiadiazol-2-yl]acetamide.

According to the present invention, the undesirable effects can be reduced by complexing the substances of pharmacological interest with cyclodextrins.

It is known that cyclodextrins are natural cyclic oligosaccharides produced by bacterial metabolism for degradation of starch (Bottoni G, Chiarabaglio G, Moro L and Rigamonti S. 4/II Il Prodotto Chimico, 1984). Three types of natural cyclodextrin are classified: α, β and γ. α cyclodextrin consists of six units, β cyclodextrin consists of seven units, and γ cyclodextrin consists of eight units. These units form hydrophobic cavities, which can contain lipophilic drugs and substances or parts thereof. In physiological solution, the complexes tend to dissolve with a kinetics that depends on the affinity of the drug for cyclodextrin. A whole series of semisynthetic cyclodextrins is now available, obtained by adding organic groups to natural cyclodextrin, varying their chemical-physical properties.

### Summary of the Invention

The aim of the invention is to provide pharmaceutical formulations aimed at treating muscular weakness caused by effort in general and in particular associated with the loss of plasma K⁺ due to loss of biological liquids or for example in individuals subjected to dialysis, muscular disorders associated with altered excitability of the tissue, such as periodic paralyses and myotonias, muscular disorders associated with muscular fatigue and tissue degeneration, such as for example Duchenne muscular dystrophy and mitochondrial myopathies, and entailing lower side effects with a better pharmacological profile than noncomplexed molecules.

This aim is achieved by the complexes according to the invention, hydroxypropyl-propyl-β-cyclodextrin + 6-[1-piperidinyl]pyrimidine-2,4-diamine-3-oxide, hydroxypropyl-propyl-β-cyclodextrin + [±]-N-cyano-N-4-pyridinyl-N-[1,2,2-trimethylpropyl]-guanidine, and hydroxypropyl-propyl-β-cyclodextrin + N-[5-sulfamoyl-1,3,4-thiadiazol-2-yl]acetamide, which perform their effect by virtue of an increased maximum duration of effort, a reduction in recovery times after effort and a consequent improvement in muscular performance, and restoration of blood K⁺ values after exercise.

An object of the present invention is to provide new pharmaceutical formulations based on complexes of cyclodextrins with pyridine-3-oxides, cyanoguanidines and thiazides, together with pharmaceutically acceptable excipients, for the treatment of muscular disorders associated with muscular weakness arising from prolonged effort and/or associated with muscular fatigue. The formulations in liquid or solid form can be used, by way of non-limitative example, as gelatins, caps, tablets, in injectable solutions or orally, in powders and granules.

These formulations can be associated with dietary supplements such as carnitine, creatine, pyruvic acid, conjugated linoleic acid, hydroxycitrate, vitamins in the treatment of muscular disorders in individuals subjected to intense physical activity.

These formulations can be associated with anorexant hormones, such as leptin, and with supplements such as chromium and selenium in the treatment of muscular weakness in obese individuals.

This aim and object are achieved by the composition comprising at least one cyclodextrin and a compound chosen from the group that consists of 6-[1-piperidinyl]pyrimidine-2,4-diamine-3-oxide, [±]-N-cyano-N-4-pyridinyl-N-[1,2,2-trimethylpropyl]-guanidine, or N-[5-sulfamoyl-1,3,4-thiadiazol-2-yl]acetamide. Preferably but in a nonlimitative manner, the cyclodextrin is hydroxypropyl-β-cyclodextrin. Complexing of these molecules with hydroxypropyl-β-cyclodextrin and with other types of cyclodextrin different from this one can lead to an improvement in the pharmacological profile of the substances in muscular disorders. Complexing of these molecules with hydrophobic cyclodextrins such as dimethyl and trimethyl cyclodextrins and other more hydrophobic cyclodextrins allows the slow release of these molecules. The advantages of microencapsulation of the three classes of drugs with cyclodextrins can be summarized in five points: 1) in the formulation of oral preparations, they improve the uniformity of content in low-dose tablets; 2) they improve the physical stability of the substances; 3) hydrophilic cyclodextrins increase the bioavailability of pyridine-3-oxides, cyanoguanidines and thiazides, which are scarcely watersoluble, reducing their minimum effective dose; 4) the use of hydrophobic cyclodextrins delays absorption of these drugs, allowing the preparation of delayed-action complexes; 5) the variation in bioavailability of the substances microencapsulated in the cyclodextrins can improve the pharmacological profile of the substances, reducing the incidence of side effects.

The use of cyclodextrins allows to improve unexpectedly and significantly the bioavailability of pyridine-3-oxides, cyanoguanidines and thiazides.

The above cited compounds are used to treat muscular weakness due to effort in general and in particular associated with the loss of plasma K⁺ due to urine loss or loss of biological liquids or for example in individuals subjected to dialysis.

The compounds described in the present invention are used to treat muscular disorders associated with altered excitability of the tissue, particularly for treating periodic paralyses and for treating myotonias.

The compounds are also used to treat muscular disorders associated with muscular fatigue and tissue degeneration, particularly Duchenne muscular dystrophy.

Finally, the compounds are used for the treatment of mitochondrial myopathies.

The complexes according to the invention are prepared with the so-called "kneading" technique. According to this method, the drug and the cyclodextrin are dissolved in a few ml of appropriate solvent so as to obtain a uniform paste. The paste is then pressed and dried until microcrystals are obtained; the microcrystals are then reduced to powder. Test for dissolution of the complexes in physiological solutions indicate better solubility of the complexes than the free forms of the noncomplexed substances.

Tests, to be considered non-limitative, for evaluating the muscular performance of Wistar Kioto male rats were conducted. Three complexes, 6-[1-piperidinyl]pyrimidine-2,4-diamine-3-oxide + hydroxypropyl-β-cyclodextrin, [±]-N-cyano-N-4-pyridinyl-N-[1,2,2-trimethylpropyl]-guanidine + hydroxypropyl-β-cyclodextrin, and N-[5-sulfamoyl-1,3,4-thiadiazol-2-yl]acetamide + hydroxypropyl-β-cyclodextrin, were compared with placebo and with the respective noncomplexed molecules. Nine groups of rats, each constituted by eight animals, were selected. The group treated with 6-[1-piperidinyl]pyrimidine-2,4-diamine-3-oxide complexed with hydroxypropyl-β-cyclodextrin at high doses was found to be the most effective as regards the muscular performance of the animals, since at lower doses than the group treated with [±]-N-cyano-N-4-pyridinyl-N-[1,2,2-trimethylpropyl]-guanidine complexed with hydroxypropyl-β-cyclodextrin and the group treated with N-[5-sulfamoyl-1,3,4-thiadiazol-2-yl]acetamide complexed with hydroxypropyl-β-cyclodextrin it produced significant improvements in muscular performance. The three complexes were found to be significantly and unexpectedly better than the respective noncomplexed molecules. The complexes according to the invention, and particularly those with 6-[1-piperidinyl]pyrimidine-2,4-diamine-3-oxide, are an important improvement from the pharmacological standpoint with respect to the noncomplexed substances, since they increase the maximum duration of the effort, reduce the time for recovery after muscular effort and therefore improve muscular performance, and restore the values of blood K⁺ after physical exercise.

Evaluation tests for the main side effects (hypotension and heart rate increase) of the three molecules were also conducted, comparing them with the respective complexes and with placebo. Generally, the complexes exhibited a trend similar to placebo, without displaying significant hypotensive effects or significant increases in heart rate. The 6-[1-piperidinyl]pyrimidine-2,4-diamine-3-oxide + hydroxypropyl-β-cyclodextrin complex proved itself particularly interesting; in this complex, the incidence of hypotension in animals was significantly lower than in the group treated with the substance in noncomplexed form. These complexes therefore reduce the incidence of the undesirable effects, which are invalidating for long-term treatments based on the use of pyridine-3-oxides, guanidines and thiazides alone, such as indeed hypotension and heart rate increase, thus allowing their chronic use.

The examples that follow are merely illustrations of the present invention and must not be understood as limiting it.

### Example 1

### Preparation of the compounds

The complexes were prepared by means of the so-called "kneading" technique described briefly hereafter. The drug and the hydroxypropyl-β-cyclodextrin were weighed in equimolecular ratios and then crushed in a mortar, gradually adding a few ml of a mixture of water and ethanol at 50%, until a uniform paste was obtained. The resulting paste was pressed for approximately 30 minutes and was then dehydrated on a stove at 50 °C for 2 days. After drying, microcrystals of the complex were obtained and were reduced to a fine powder in a mortar.

### Example 2

### Pharmacological tests

The effectiveness of the complexes was compared with the effectiveness of the respective noncomplexed drugs.

The following complexes were compared:
complex 1 = 6-[1-piperidinyl]pyrimidine-2,4-diamine-3-oxide + hydroxypropyl-β-cyclodextrin;
complex 2 = [±]-N-cyano-N-4-pyridinyl-N-[1,2,2-trimethylpropyl]-guanidine + hydroxypropyl-β-cyclodextrin;
complex 3 = N-[5-sulfamoyl-1,3,4-thiadiazol-2-yl]acetamide + hydroxypropyl-β-cyclodextrin.

Wistar Kioto male rats weighing 350 g were divided into nine groups of eight animals each (numbered 1 to 8 for each test group) and were treated with amounts of complex 1 equal to 20 µg/kg/day or 40 µg/kg/day of noncomplexed active ingredient, of complex 2 equal to 0.4 mg/kg/day of noncomplexed active ingredient and of complex 3 equal to 6 mg/kg/day of noncomplexed active ingredient, orally with an endogastric tube for 30 days, during which the animals were exercised and tested. The tested doses were the minimum ones required to restore blood K⁺ levels after exercise.
Placebo group treated with physiological solution enriched with hydroxypropyl-β-cyclodextrin;
Group treated with 20 µg/kg/day of 6-[1-piperidinyl]pyrimidine-2,4-diamine-3-oxide;
Group treated with 40 µg/kg/day of 6-[1-piperidinyl]pyrimidine-2,4-diamine-3-oxide;
Group treated with 180 µg/kg/day of complex 1;
Group treated with 360 µg/kg/day of complex 1;
Group treated with 0.4 mg/kg/day of [±]-N-cyano-N-4-pyridinyl-N-[1,2,2-trimethylpropyl]-guanidine;
Group treated with 3 mg/kg/day of complex 2;
Group treated with 6 mg/kg/day of N-[5-sulfamoyl-1,3,4-thiadiazol-2-yl]acetamide;
Group treated with 50 mg/kg/day of complex 3.

These animals were subjected to evaluation of the average duration of effort on a treadmill (AD, Instrument USA) during 30 days of exercise every 7 days, and average recovery times after exercise were measured on day 30 of treatment and are defined as the minimum times required in order to repeat an effort comparable, in terms of duration and intensity, with the one made at that day of treatment. Levels of venous plasma K⁺ taken from the femoral vein before and after exercise were also measured once a week per rat in animals anesthetized with chloral hydrate at a dose of 0.5 g/kg.

Each test group was subjected to daily trials at an initial speed of 15 m/min. In the second week, the speed was increased to 25 m/min, in the third week it was raised to 35 m/min, and in the fourth week it was increased to 45 m/min. Administration was performed in the morning.

The treadmill was equipped with a sensor which was sensitive to the weight of the rat, set to a generated weight force of 60 g/30 sec, and inserted in the rear wall of the time measurement chamber.

### Results of groups treated with 6-[1-piperidinyl]pyrimidine-2,4-diamine-3-oxide

Placebo exhibited a bell-curve trend, with peaks of exercise duration around day 14 of treatment, gradually decreasing thereafter to values lower than those measured before the treatment (Chart 1). The group treated with complex 1 and the group treated with noncomplexed 6-[1-piperidinyl]pyrimidine-2,4-diamine-3-oxide exhibited a trend similar to placebo up to day 14 of treatment, but distinguished themselves from the placebo after day 14; after this, average duration of the effort on the treadmill remained significantly longer than in the placebo group. No significant differences were noted between the group treated with noncomplexed 6-[1-piperidinyl]pyrimidine-2,4-diamine-3-oxide at high dosage and the group treated with complex 1 at high dosage. These effects were observed in all the animals treated with noncomplexed 6-[1-piperidinyl]pyrimidine-2,4-diamine-3-oxide or with the form complexed with cyclodextrin (Chart 1).

### Chart 1

### Trend of duration of effort as a function of treatment with 6-[1-piperidinyl]pyrimidine-2,4-diamine-3-oxide (40 µg/kg/day) and complex 1 (360 µg/kg/day)

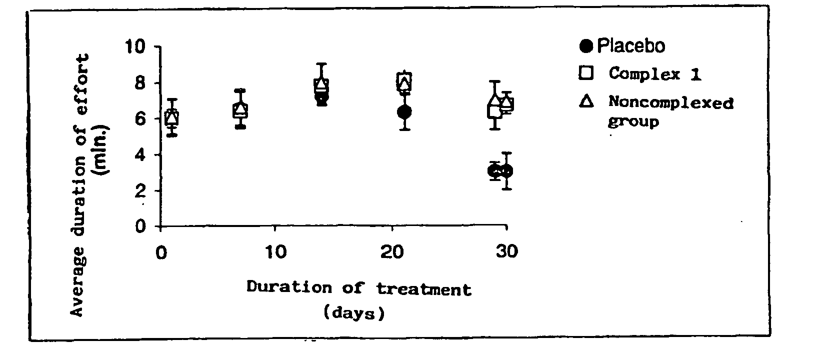

Average recovery times after effort, measured as times required to repeat the performance sustained at that given day of treatment, were reduced significantly with respect to placebo. At day 30 of treatment, 6 rats out of 8 of the group treated with 6-[1-piperidinyl]pyrimidine-2,4-diamine-3-oxide were in fact able to sustain an effort lasting 7.2±1 minutes on a treadmill already 30 minutes after suspending their exercise; the remaining 2 rats were able to do so only after 90 minutes and did not differ from placebo. For the group treated with complex 1, too, average recovery times after effort were reduced significantly with respect to placebo. For this group, after 30 days of treatment, 7 rats out of 8 were able to sustain an effort lasting 7.5±1 minutes on a treadmill already after 30 minutes and 1 rat was able to do so after 110 minutes and did not differ from placebo. For the group treated with placebo, the times required in order to sustain an effort lasting 6.2±1 minutes comparable to the one sustained earlier were longer than 100 minutes for all the animals.

Levels of blood K⁺ measured before the effort in the morning, prior to administration at rest, were 4.5±0.8 mEq/l in the placebo group and between 4 mEq/l and 5.5 mEq/l for groups treated with 6-[1-piperidinyl]pyrimidine-2,4-diamine-3-oxide in free or complexed form. 30 minutes after the end of the effort, the values of K⁺ were 3.1±2 mEq/l in the placebo group, 5.2±2 mEq/l for the group treated with 6-[1-piperidinyl]pyrimidine-2,4-diamine-3-oxide, and 6.3±2.1 mEq/l for the group treated with complex 1; after 60 minutes, the values were 2.6±1 mEq/l in the placebo group, 4.2±1 mEq/l for the group treated with 6-[1-piperidinyl]pyrimidine-2,4-diamine-3-oxide, and 5.3±1 mEq/l for the group treated with complex 1. In accordance with previous findings (Sejersted and Sjogaard, Physiol. Rev. 1411, 2000), also in our experiments the levels of blood K⁺ return to rest values after exercise; after 100 minutes, the levels of venous K⁺ were in fact 5.2±1 mEq/l in the placebo group, 4.5±1 mEq/l for the group treated with 6-[1-piperidinyl]pyrimidine-2,4-diamine-3-oxide, and 5.1±1 mEq/l for the group treated with complex 1.

A similar trend was achieved with the group treated with 6-[1-piperidinyl]pyrimidine-2,4-diamine-3-oxide complexed with cyclodextrin at the dose of 180 µg/kg/day and with the noncomplexed group at the dose of 20 µg/kg/day, but a smaller number of animals responded; in both groups, 3 rats out of 8 in fact responded to the treatment in terms of both functional and blood parameters and the remainder behaved like the placebo group. The 5 animals per test group that did not respond to the treatment were unable to repeat the effort and also showed K⁺ levels lower than 3 mEq/l up to 100 minutes after the end of the effort.

### Results of groups treated with [±]-N-cyano-N-4-pyridinyl-N-[1,2,2-trimethylpropyl]-guanidine

The group treated with complex 2 and the group treated with noncomplexed [±]-N-cyano-N-4-pyridinyl-N-[1,2,2-trimethylpropyl]-guanidine exhibited a trend similar to placebo up to day 14 of treatment, but distinguished themselves from the placebo after day 14, after which the average duration of the effort on the treadmill remained significantly longer (Chart 2). No significant differences were noted between the group treated with [±]-N-cyano-N-4-pyridinyl-N-[1,2,2-trimethylpropyl]-guanidine and the group treated with complex 2.

### Chart 2

### Trend of duration of effort as a function of treatment with [±]-N-cyano-N-4-pyridinyl-N-[1,2,2-trimethylpropyl]-guanidine (0.4 mg/kg/day) and complex 2 (3 mg/kg/day)

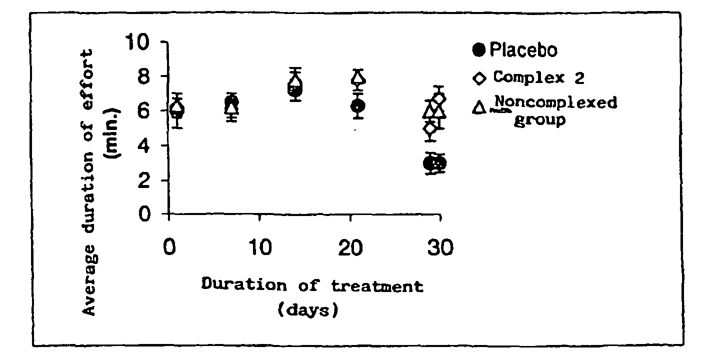

Average recovery times after effort, measured as times required to repeat the performance sustained earlier at that given day of treatment, were reduced significantly with respect to the placebo. After thirty days of treatment, 5 rats out of 8 of the group treated with [±]-N-cyano-N-4-pyridinyl-N-[1,2,2-trimethylpropyl]-guanidine were in fact able to sustain an effort lasting 7.4±1 minutes on a treadmill already 30 minutes after the end of the exercise, and the remaining 3 rats were able to do so after 70 minutes. For the group treated with complex 2, also, average recovery times after effort were reduced significantly with respect to the placebo; after thirty days of treatment, 6 rats out of 8 were in fact able to sustain an effort lasting 8.1±1 minutes on a treadmill already 30 minutes after the end of the exercise and the remaining rats were able to do so after 80 minutes.

Levels of blood K⁺ measured before the effort in the morning, prior to administration, were 4.5±0.8 mEq/l in the placebo and between 5 mEq/l and 5.5 mEq/l for the group treated with noncomplexed [±]-N-cyano-N-4-pyridinyl-N-[1,2,2-trimethylpropyl]-guanidine and the group treated with complex 2. In the animals that responded to treatment, 30 minutes after the end of the administration the K⁺ values were 5.2±2 mEq/l for the group treated with noncomplexed [±]-N-cyano-N-4-pyridinyl-N-[1,2,2-trimethylpropyl]-guanidine and 6.3±2.1 for the group treated with complex 2; after 60 minutes, the values were 4.8±1 mEq/l for the group treated with noncomplexed [±]-N-cyano-N-4-pyridinyl-N-[1,2,2-trimethylpropyl]-guanidine and 5.6±1 mEq/l for the group treated with complex 2; after 100 minutes, the levels of venous K⁺ were 4.6±1 mEq/l for the group treated with noncomplexed [±]-N-cyano-N-4-pyridinyl-N-[1,2,2-trimethylpropyl]-guanidine and 4.8±1 mEq/l for the group treated with complex 2. The animals of both test groups that did not respond to treatment were unable to repeat the effort and also exhibited levels of K⁺ below 3 mEq/l up to 100 minutes after the end of the effort.

### Results of groups treated with N-[5-sulfamoyl-1,3,4-thiadiazol-2-yl]acetamide

The group treated with complex 3 and the group treated with noncomplexed N-[5-sulfamoyl-1,3,4-thiadiazol-2-yl]acetamide exhibited a trend similar to placebo up to day 14 of treatment but distinguished themselves from the placebo after day 14, after which the average duration of the effort on the treadmill remained significantly longer. No significant differences were observed between the group treated with N-[5-sulfamoyl-1,3,4-thiadiazol-2-yl]acetamide and the group treated with complex 3.

### Chart 3

### Trend of duration of effort as a function of treatment with N-[5-sulfamoyl-1,3,4-thiadinol-2-yl]acetamide (6 mg/kg/day) and complex 3 (50 mg/kg/day)

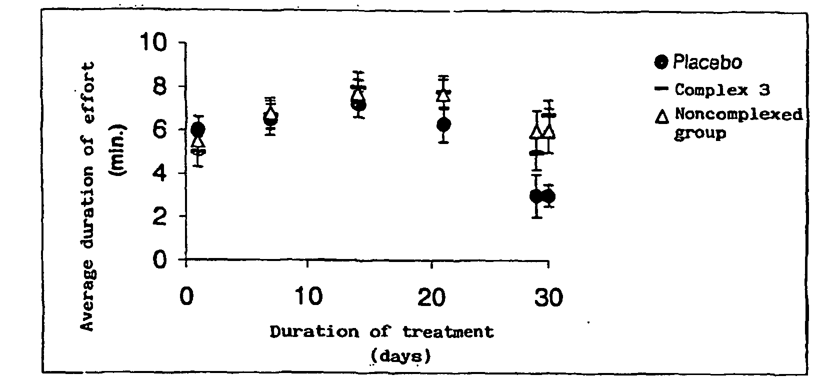

Average recovery times after effort, measured as times required to repeat the performance sustained earlier at that given day of treatment, were reduced significantly with respect to placebo. After thirty days of treatment, 4 rats out of 8 of the group treated with N-[5-sulfamoyl-1,3,4-thiadiazol-2-yl]acetamide were in fact able to sustain an effort lasting 7±2 minutes on a treadmill already 30 minutes after the end of the exercise, and the remaining 4 rats were able to do so after 80 minutes. For the group treated with complex 3, also, after thirty days of treatment 6 rats out of 8 were able to sustain an effort lasting 7.4±1 minutes on a treadmill already 30 minutes after the end of the exercise and the remaining 2 rats were able to do so after 70 minutes.

Levels of blood K⁺ measured before the effort in the morning, prior to administration, were between 4.5 mEq/l and 5 mEq/l for the group treated with noncomplexed N-[5-sulfamoyl-1,3,4-thiadiazol-2-yl]acetamide and for the group treated with complex 3. In the rats that responded to treatment, 30 minutes after the end of the administration the K⁺ values were 6.2±2 mEq/l for the group treated with N-[5-sulfamoyl-1,3,4-thiadiazol-2-yl]acetamide and 7.3±2.1 for the group treated with complex 3; after 60 minutes, the values were 5.8±1 mEq/l for the group treated with N-[5-sulfamoyl-1,3,4-thiadiazol-2-yl]acetamide and 6.6±1 mEq/l for the group treated with complex 3; after 100 minutes, the levels of venous K⁺ were 5.6±1 mEq/l for the group treated with N-[5-sulfamoyl-1,3,4-thiadiazol-2-yl]acetamide and 5.8±1 mEq/l for the group treated with complex 3. The animals of both test groups that did not respond to treatment were unable to repeat the effort and also exhibited levels of K⁺ below 3 mEq/l up to 100 minutes after the end of the effort.

### Example 3

### Evaluation of undesirable effects

### HYPOTENSION

Pressure measurements were made by means of a T206 dual-channel flow meter by Transonic System Inc (Ithaca, NY, USA). In the control animals not subjected to stress or pharmacological treatments, pressure was 91±7 mm Hg (N=56 rats). The pressure values of the animals in our test groups prior to the beginning of pharmacological treatment were between 85 mm Hg and 105 mm Hg. The pressures were measured in the morning, at rest, prior to administration. Data are expressed as percentage variations with respect to the pressure that each animal exhibited in the morning, before starting the pharmacological treatment. The group treated with noncomplexed 6-[1-piperidinyl]pyrimidine-2,4-diamine-3-oxide exhibited a slight reduction in average pressure by 8%, which was more conspicuous in the group treated with [±]-N-cyano-N-4-pyridinyl-N-[1,2,2-trimethylpropyl]-guanidine and in the group treated with N-[5-sulfamoyl-1,3,4-thiadiazol-2-yl]acetamide, where the reduction was respectively 13% and 15%. The observed variations remained constant to the end of the treatment. Before the 21^{st} day, no significant pressure variations were observed for all test groups. The pressure values used to plot the chart refer to the values from day 21 to day 30 of treatment (Chart 4).

### Chart 4

### Trend of pressure variations as a function of treatment with 6-[1-piperidinyl]pyrimidine-2,4-diamine-3-oxide (40 µg/kg/day), [±]-N-cyano-N-4-pyridinyl-N-[1,2,2-trimethylpropyl]-guanidine (0.4 mg/kg/day), N-[5-sulfamoyl-1,3,4-thiadiazol-2-yl]acetamide (6 mg/kg/day)

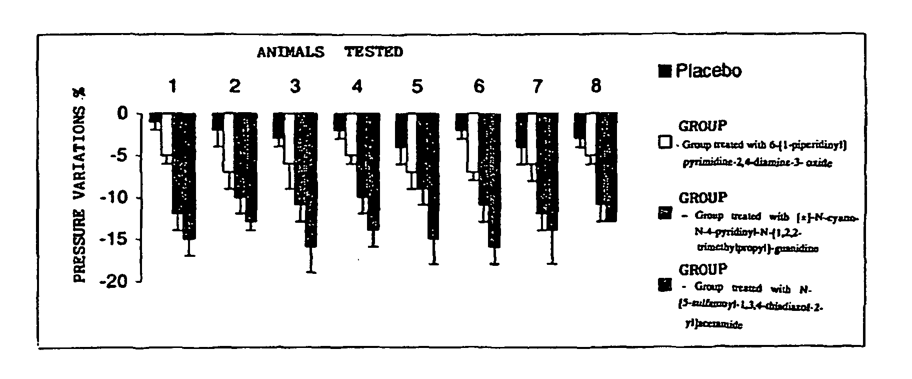

The complexes with cyclodextrin exhibited a trend similar to placebo, without significant hypotensive effects, particularly for the group treated with complex 1 (360 µg/kg/day), where 8 rats out of 8 did not show hypotension, and partly for the group treated with complex 2 (3 mg/kg/day) in which 6 rats out of 8 showed no hypotension, while 2 rats showed a pressure drop of more than 5%. For the group treated with complex 3 (50 mg/kg/day), 5 rats out of 8 showed no significant hypotension, while in the remaining 3 the pressure drop was more than 5%. Up to day 21, no significant pressure variations were observed for all test groups. The pressure values used to plot the chart refer to the values from day 21 to day 30 of treatment. (Chart 5)

### Chart 5

### Trend of pressure variations as a function of treatment with complex 1 (360 µg/kg/day), complex 2 (3 mg/kg/day), and complex 3 (50 mg/kg/day)

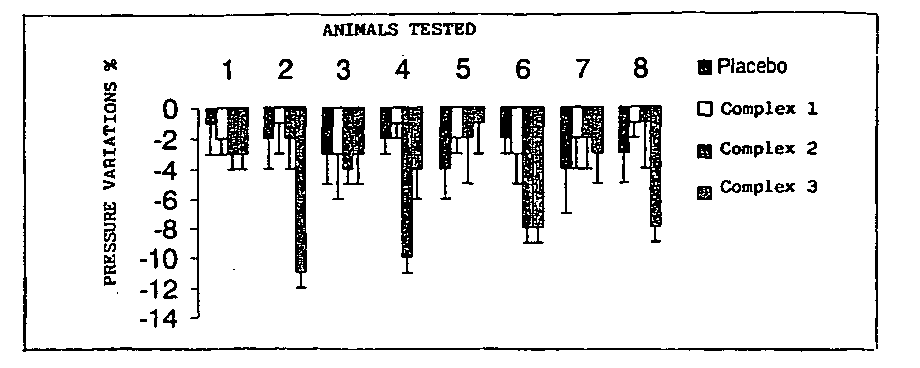

### HEART RATE

The average heart rate (HR) in control animals not subjected to stress or pharmacological treatments was 295±18 bpm (N = 56 rats). The HR values of the animals before pharmacological treatment began were between 275 and 302 bpm. Rates were measured in the morning, at rest, prior to administration. Data are expressed as percentage variation with respect to the values of the rates exhibited by each animal in the morning before pharmacological treatment began. The group treated with noncomplexed 6-[1-piperidinyl]pyrimidine-2,4-diamine-3-oxide, from day 21 of treatment onward, exhibited an average rate increase of 10±2%; the group treated with [±]-N-cyano-N-4-pyridinyl-N-[1,2,2-trimethylpropyl]-guanidine had an increase of 14±2%; and the group treated with N-[5-sulfamoyl-1,3,4-thiadiazol-2-yl]acetamide had an increase of 9±2%.

The complexes with cyclodextrin exhibited a trend similar to placebo, showing no significant increases in HR; in particular, for the group treated with complex 1 (360 µg/kg/day), 8 rats out of 8 showed no increase in heart rate up to the end of the treatment. For the group treated with complex 2 (3 mg/kg/day), 2 rats out of 8 showed a heart rate increase of 6±2%; these animals were also the ones that exhibited hypotension. For the group treated with complex 3 (50 mg/kg/day), 3 rats out of 8 exhibited an increase in HR of 8±3% and were the ones that exhibited hypotension.

## Claims

1. A pharmaceutical composition, **characterized in that** it comprises at least one cyclodextrin complexed with a compound chosen from the group that consists of 6-[1-piperidinyl]pyrimidine-2,4-diamine-3-oxide, [±]-N-cyano-N-4-pyridinyl-N-[1,2,2-trimethylpropyl]-guanidine, or N-[5-sulfamoyl-1,3,4-thiadiazol-2-yl]acetamide for the treatment of muscular disorders.

2. The composition according to claim 1, **characterized in that** the cyclodextrin is hydroxypropyl-β-cyclodextrin.

3. Use of the composition according to claim 1 or 2 to produce a pharmaceutical formulation for treatment of effort-related muscular weakness.

4. Use of the composition according to claim 1 or 2 to produce a pharmaceutical formulation for the treatment of neuromuscular disorders associated with altered excitability of tissue.

5. Use according to claim 4 wherein said disorder is periodic paralyses.

6. Use according to claim 4 wherein said disorder is myotonias.

7. Use of the composition according to claim 1 or 2 to produce a pharmaceutical formulation for the treatment of muscular disorders associated with muscular fatigue and tissue degeneration.

8. Use according to claim 7 wherein said disorder is Duchenne muscular dystrophy.

9. Use of the composition according to claim 1 or 2 to produce a pharmaceutical formulation for the treatment of mitochondrial myopathies.

10. Use of cyclodextrin to produce a preparation for improving the oral bioavailability of 6-[1-piperidinyl]pyrimidine-2,4-diamine-3-oxide, [±]-N-cyano-N-4-pyridinyl-N-[1,2,2-trimethylpropyl]-guanidine, N-[5-sulfamoyl-1,3,4-thiadiazol-2-yl]acetamide.

11. The use of cyclodextrins according to claim 10, wherein the cyclodextrins are hydrophilic and particularly hydroxypropyl-β-cyclodextrin.

12. Use of hydrophobic cyclodextrins to produce a preparation for the slow release of 6-[1-piperidinyl]pyrimidine-2,4-diamine-3-oxide, [±]-N-cyano-N-4-pyridinyl-N-[1,2,2-trimethylpropyl]-guanidine, N-[5-sulfamoyl-1,3,4-thiadiazol-2-yl]acetamide.

13. A pharmaceutical formulation, **characterized in that** it comprises the composition according to claim 1 with pharmaceutically acceptable excipients.

14. A pharmaceutical formulation, **characterized in that** it comprises the composition according to claim 1 and comprising at least one or more of the following dietary supplements: carnitine, creatine, pyruvic acid, conjugated linoleic acid, vitamins, hydroxycitrate, chrome, selenium, and/or anorexant hormones.

15. A pharmaceutical formulation according to claim 14, wherein at least one of the anorexant hormones is leptin.

16. The pharmaceutical formulation according to claim 14 or 15 in the form of a preparation to be administered orally, in the form of capsules or gel.

## Patentansprüche

1. Pharmazeutische Zusammensetzung, **dadurch gekennzeichnet, dass** sie mindestens ein Cyclodextrin umfasst, das einen Komplex mit einer Verbindung bildet, die aus der Gruppe ausgewählt wird, die aus 6-[1-Piperidinyl]pyrimidin-2,4-diamin-3-oxid, [±]-N-Cyano-N-4-pyridinyl-N-[1,2,2-trimethylpropyl]-guanidin oder N-[5-Sulfamoyl-1,3,4-thiadiazol-2-yl]acetamid besteht, zur Behandlung von Muskelstörungen.

2. Zusammensetzung nach Anspruch 1, **dadurch gekennzeichnet, dass** das Cyclodextrin Hydroxypropyl-β-cyclodextrin ist.

3. Verwendung der Zusammensetzung nach Anspruch 1 oder 2 für die Herstellung einer pharmazeutischen Formulierung zur Behandlung von belastungsabhängiger Muskelschwäche.

4. Verwendung der Zusammensetzung nach Anspruch 1 oder 2 für die Herstellung einer pharmazeutischen Formulierung zur Behandlung von neuromuskulären Störungen, die mit veränderter Gewebeerregbarkeit assoziiert sind.

5. Verwendung nach Anspruch 4, wobei es sich bei der Störung um periodische Paralyse handelt.

6. Verwendung nach Anspruch 4, wobei es sich bei der Störung um Myotonie handelt.

7. Verwendung der Zusammensetzung nach Anspruch 1 oder 2 für die Herstellung einer pharmazeutischen Formulierung zur Behandlung von Muskelstörungen, die mit Muskelermüdung und Gewebedegeneration assoziiert sind.

8. Verwendung nach Anspruch 7, wobei es sich bei der Störung um Muskeldystrophie vom Typ Duchenne handelt.

9. Verwendung der Zusammensetzung nach Anspruch 1 oder 2 für die Herstellung einer pharmazeutischen Formulierung zur Behandlung von mitochondrialer Myopathie.

10. Verwendung von Cyclodextrin für die Herstellung eines Präparats zur Verbesserung der oralen Bioverfügbarkeit von 6-[1-Piperidinyl]pyrimidin-2,4-diamin-3-oxid, [±]-N-Cyano-N-4-pyridinyl-N-[1,2,2-trimethylpropyl]-guanidin, N-[5-Sulfamoyl-1,3,4-thiadiazol-2-yl]acetamid.

11. Verwendung von Cyclodextrinen nach Anspruch 10, wobei die Cyclodextrine hydrophil sind und es sich insbesondere um Hydroxypropyl-β-cyclodextrin handelt.

12. Verwendung von hydrophoben Cyclodextrinen für die Herstellung eines Präparats zur langsamen Freisetzung von 6-[1-Piperidinyl]pyrimidin-2,4-diamin-3-oxid, [±]-N-Cyano-N-4-pyridinyl-N-[1,2,2-trimethylpropyl]-guanidin, N-[5-Sulfamoyl-1,3,4-thiadiazol-2-yl]acetamid.

13. Pharmazeutische Formulierung, **dadurch gekennzeichnet, dass** sie die Zusammensetzung nach Anspruch 1 mit pharmazeutisch zulässigen Trägerstoffen umfasst.

14. Pharmazeutische Formulierung, **dadurch gekennzeichnet, dass** sie die Zusammensetzung nach Anspruch 1 und mindestens einen oder mehr der folgenden Nahrungsergänzungsstoffe umfasst: Carnitin, Creatin, Benztraubensäure, konjugierte Linolsäure, Vitamine, Hydroxycitrat, Chrom, Selen und/oder Appetitzügler-Hormone.

15. Pharmazeutische Formulierung nach Anspruch 14, wobei mindestens eines der Appetitzügler-Hormone Leptin ist.

16. Pharmazeutische Formulierung nach Anspruch 14 oder 15 in Form eines oral zu verabreichenden Präparats, in Form von Kapseln oder in Gelform.

## Revendications

1. Composition pharmaceutique,
**caractérisée en ce qu'**elle comprend au moins une cyclodextrine complexée avec un composé choisi parmi le groupe qui se compose de 6-[1-pipéridinyl]pyrimidine-2,4-diamine-3-oxyde, [±]-N-cyano-N-4-pyridinyl-N-[1,2,2-triméthylpropyl]-guanidine ou N-[5-sulfamoyl-1,3,4-thiadiazol-2-yl]acétamide pour le traitement de troubles musculaires.

2. Composition selon la revendication 1,
**caractérisée en ce que** la cyclodextrine est l'hydroxypropyl-β-cyclodextrine.

3. Utilisation de la composition selon la revendication 1 ou 2, pour produire une formulation pharmaceutique pour le traitement de faiblesses musculaires relatives à l'effort.

4. Utilisation de la composition selon la revendication 1 ou 2, pour produire une formulation pharmaceutique pour le traitement de troubles neuromusculaires associés à l'excitabilité de tissu modifiée.

5. Utilisation selon la revendication 4, dans laquelle ledit trouble est une paralysie périodique.

6. Utilisation selon la revendication 4, dans laquelle ledit trouble est une myotonie.

7. Utilisation de la composition selon la revendication 1 ou 2, pour produire une formulation pharmaceutique pour le traitement de troubles musculaires associés à la fatigue musculaire et à la dégénérescence tissulaire.

8. Utilisation selon la revendication 7, dans laquelle ledit trouble est la dystrophie musculaire de Duchenne.

9. Utilisation de la composition selon la revendication 1 ou 2, pour produire une formulation pharmaceutique pour le traitement de myopathies congénitales mitochondriales.

10. Utilisation de cyclodextrine pour produire une préparation pour améliorer la biodisponibilité orale de 6-[1-pipéridinyl]pyrimidine-2,4-diamine-3-oxyde, [±]-N-cyano-N-4-pyridinyl-N-[1,2,2-triméthylpropyl]-guanidine, N-[5-sulfamoyl-1,3,4-thiadiazol-2-yl]acétamide.

11. L'utilisation de cyclodextrines selon la revendication 10, dans laquelle les cyclodextrines sont hydrophiles et particulièrement l'hydroxypropyl-β-cyclodextrine.

12. Utilisation de cyclodextrines hydrophobes pour produire une préparation pour la libération lente de 6-[1-pipéridinyl]pyrimidine-2,4-diamine-3-oxyde, [±]-N-cyano-N-4-pyridinyl-N-[1,2,2-triméthylpropyl]-guanidine, N-[5-sulfamoyl-1,3,4-thiadiazol-2-yl]acétamide.

13. Formulation pharmaceutique,
**caractérisée en ce qu'**elle comprend la composition selon la revendication 1 avec des excipients pharmaceutiquement acceptables.

14. Formulation pharmaceutique,
**caractérisée en ce qu'**elle comprend la composition selon la revendication 1 et comprenant au moins un ou plusieurs des suppléments diététiques suivants : la carnitine, la créatine, l'acide pyruvique, l'acide linoléique conjugué, les vitamines, l'hydroxycitrate, le chrome, le sélénium et/ou les hormones anorexantes.

15. Formulation pharmaceutique selon la revendication 14, dans laquelle au moins l'une des hormones anorexantes est la leptine.

16. Formulation pharmaceutique selon la revendication 14 ou 15 sous la forme d'une préparation à administrer oralement, sous la forme de gélules ou de gel.
